# EUROPEAN PATENT SPECIFICATION

(11) **EP 3 952 974 B1**
(45) Date of publication and mention of the grant of the patent: **01.10.2025**
(21) Application number: 20753492.6
(22) Date of filing: 15.06.2020
(51) Int. Cl.: A61M 39/24, A61M 27/00, A61M 25/00, A61M 39/28

(54) **URINARY CATHETER DRAINAGE MEMBERS THAT RESTRICT FLUID DRAINAGE**
URINKATHETERDRAINAGEELEMENTE MIT BEGRENZUNG DER FLÜSSIGKEITSDRAINAGE
ÉLÉMENTS DE DRAINAGE DE CATHÉTER URINAIRE QUI RESTREIGNENT LE DRAINAGE DE FLUIDE

(30) Priority: 08.07.2019 US 201962871512 P
(43) Date of publication of application: 16.02.2022
(73) Proprietor: Hollister Incorporated, Libertyville, IL 60048-3781 (US)
(72) Inventor: O'BRIEN, Daniel E., Libertyville, IL 60048 (US); NAUGHTON, Vincent, Libertyville, IL 60048 (US); KNAUZ, David A., Libertyville, IL 60048 (US)
(74) Representative: FRKelly
(86) International application number: PCT/US2020/037694
(87) International publication number: WO 2021/006994

(56) References cited:
- WO-A1-99/45997
- US-A- 4 611 785
- US-A- 4 932 938
- US-A- 4 946 449
- US-A1- 2013 324 975

## Description

The present application claims the benefit or and priority to U.S. Provisional Application No. 62/871,512, filed July 8, 2019.

### Background

### Field of the Disclosure

The present disclosure generally relates to urinary catheters. More particularly, the present disclosure relates to urinary catheter drainage members that selectively restrict the flow of urine or drainage of urine from the catheter as desired by the user.

### Description of Related Art

Catheters are used to treat many different types of medical conditions and typically include an elongated shaft that is inserted into and through a passageway or lumen of the body. Catheters, and in particular intermittent catheters, are commonly used by those who suffer from various abnormalities of the urinary system, such as urinary incontinence. Urinary catheters generally comprise a shaft portion with two ends. A first end has a catheter tip that is inserted into a user's urethra. A second end generally has a drainage member that is used to help facilitate and direct urine drainage.

Because urinary catheter drainage members are meant to be used by individuals with limited dexterity, it is desirable for them to be easily manipulated in order to control the flow of urine being drained by the catheter. One challenge that may be encountered with drainage members currently known in the prior art is that flow of urine may exit through the drainage members too quickly and uncontrollably. This may be the case when the user initially inserts the catheter. Commonly, the catheter user advances the catheter until urine begins to drain from the catheter. Because the user is unaware of when drainage will commence, the user may not be prepared when drainage commences. This can cause the draining urine to leak or splatter, resulting in a messy and embarrassing experience for the user. Accordingly, there is a need for improved drainage members. An example of urinary catheter drainage members are known from US2013/324975 A1 and US4611785 A.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a urinary catheter comprising a drainage member according to claim 1 is disclosed.

### Brief Description of the Drawings

Fig 1. is a side view of an embodiment of a urinary catheter showing a catheter shaft connected to a drainage member;
Fig. 2A is a perspective view of an embodiment of a drainage member of the present disclosure having a weakened portion;
Fig. 2B is a perspective view of an embodiment of a drainage member of the present disclosure having a weakened portion;
Fig. 3A is a perspective view of an embodiment of a drainage member with a generally oval shaped distal portion;
Fig. 3B is a perspective view of the drainage member of Fig. 3A;
Fig. 4A is a perspective view of an embodiment of a drainage member with a distal portion shaped in a truncated pyramid;
Fig. 4B is a perspective view of the drainage member of Fig. 4A;
Fig. 5A is a cross-sectional view of an embodiment of a drainage member with a valve located within the lumen of the catheter drainage member;
Fig. 5B is an enlarged cross-sectional view of the drainage member of Fig. 5A, showing the details of the valve;
Fig. 6A is a cross-sectional view of an embodiment of a drainage member with a valve located in the lumen of the drainage member;
Fig. 6B is an enlarged cross-sectional view of the valve shown in Fig. 6A;
Fig. 7 is a perspective view of an embodiment of a drainage member having an L-shape configuration;
Fig. 8A is a cross-sectional view of an embodiment of a drainage member with a rigid proximal portion and a flexible distal portion; and
Fig. 8B is a perspective view of the drainage member of 8A.

### Description of the Illustrated Embodiments

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

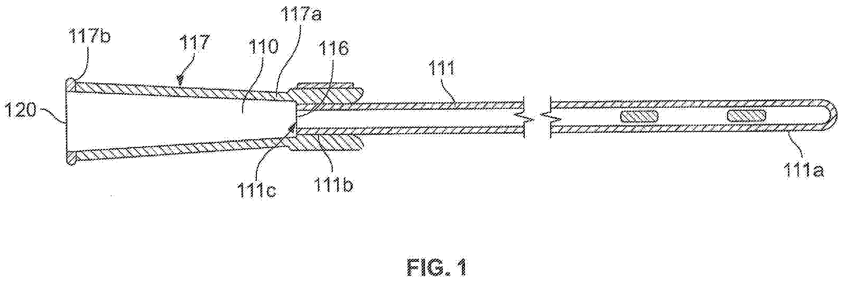

Urinary catheter drainage member configurations according to the present disclosure and their individual components may be variously configured without departing from the scope of the present disclosure, but in one embodiment, a urinary catheter drainage member is configured as shown in Fig. 1.

Fig. 1 shows an embodiment of a urinary catheter drainage member 110 connected to a catheter shaft 111. The drainage member 110 includes a body 117 having a proximal end 117a and a distal end 117b. The catheter shaft 111 includes a proximal portion 111a, a distal portion 111b, and a drainage lumen 111c. The drainage member 110 includes a lumen 116 that is fluidically connected to the catheter drainage lumen 111c. This connection allows fluid flowing into the catheter 111 to drain into the drainage member 110. The distal end 117b of the drainage member 110 has an opening 120 configured to allow fluid, that passes from the catheter 111 into the drainage member 110, to drain from the drainage member 110.

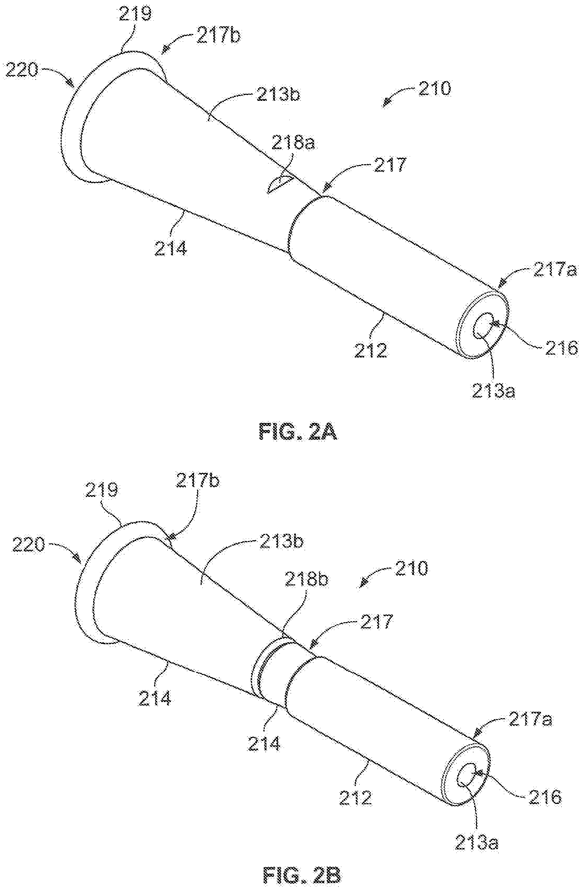

Fig. 2A shows an embodiment of a urinary catheter drainage member 210. The drainage member 210 includes a body 217. Although the illustrated body 217 has a generally round cross-section, the cross-section of the body may have other shapes, including but not limited to rectangular, oval, etc. The body 217 has a proximal portion 212 and a distal portion 214 positioned adjacent to and distal from the proximal portion 212. The body 217 also includes a proximal end 217a and a distal end 217b. The body 217 also includes an inner surface 213a and an outer surface 213b. The inner surface 213a defines a lumen 216 extending from the proximal end 217a of the body 217 through an opening 220, for urine drainage, defined by the distal end 217b of the body 217. As shown in Fig. 2A the opening 220 may be substantially circular in shape and, optionally, may be surrounded by a lip 219. The proximal end 217a is configured to be located at the distal end of a conduit (such as the distal portion 111b/drainage lumen 111c of the urinary catheter 111 shown in Fig. 1), such that the lumen 216 of the body 217 is in fluid communication with the conduit. The drainage member of Fig. 2A allows fluid to flow into the proximal end 217a of the body 217 from a conduit (for example, a urinary catheter) and out of the opening 220 defined by the distal end 217b of the body 217.

The body 217 may be comprised of flexible material. The flexible material may include an elastomer, rubber, PVC or any other suitable material which may be manipulated by a user.

In the embodiment shown in Fig. 2A, the drainage member 210 includes a weakened portion for bending and/or kinking the drainage member 210. The weakened portion allows for folding the distal portion 214 toward the proximal portion 212 and/or twisting the distal portion 214 relative to the proximal portion 212. In the illustrated embodiment, the drainage member 210 includes a notch 218a in the outer surface 213b of the body 217. The notch 218a provides a weakened portion of the body that allows the body 217 to be bent or kinked to restrict urine drainage. Restricting urine drainage includes slowing drainage and/or stopping drainage. The notch 218a may be any suitably shaped notch. In the illustrated embodiment notch 218a may be shaped like a semi-circle as shown in Fig. 2A. Furthermore, the notch may be located at any location on the body. The notch may be located at a location proximal the distal end 217b or opening 220 such that there is a sufficient amount of body to be kinked or bent to restrict the flow of urine. In one embodiment, it may be located in the middle or substantially the middle of the body. Though one notch is shown in Fig. 2A, a plurality of notches may be used. Thus, the body 217 is configured to selectively restrict urine drainage from the opening 220.

The embodiment shown in Fig. 2B has similar features to that of the embodiment shown in Fig. 2A, except that the notch 218b comprises a groove, extending at least partially around the body. The groove may extend any length around the body. In one embodiment the groove may be a groove that extends around the entire body. In the illustrated embodiment, the groove is a circumferential groove. The notch (218a, 218b) may have any other shape sufficient to provide a weakened portion to allow bending or kinking of the body, as described above.

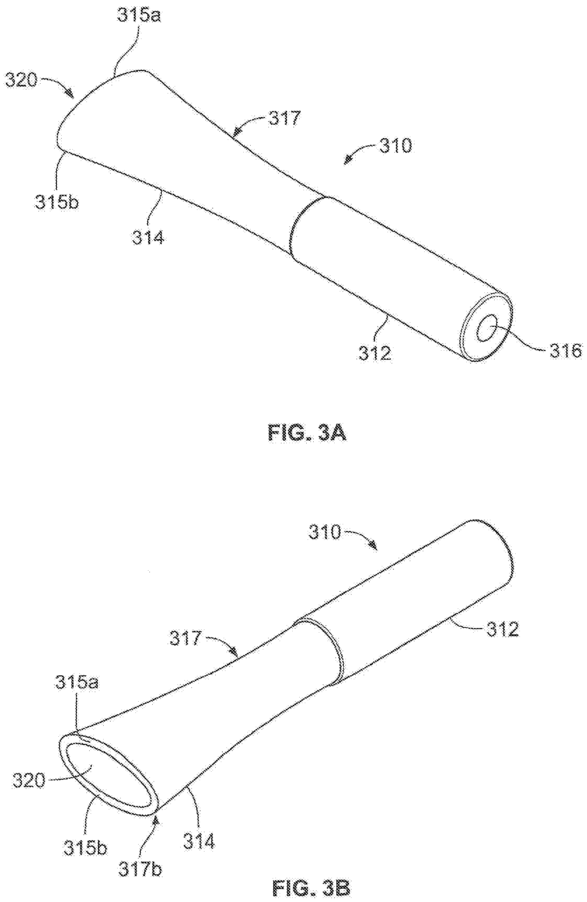

Figs. 3A and 3B show an embodiment of the drainage member 310 wherein the distal portion 314 of the body 317 includes a generally oval shape defining a top wall 315a and a bottom wall 315b. Fig. 3A shows the drainage member 310 with the proximal portion 312 in the foreground. The lumen 316 is visible. Fig. 3B shows the drainage member of Fig. 3A with the distal portion 314 in the foreground. The opening 320 is visible.

In the embodiment shown in Figs. 3A and 3B, the distal portion 314 is made from flexible material and is configured to be pinched or bent by an external stimulus, including the force of a user's fingers, in order to narrow the opening 320. For example, a user may place a thumb over the top wall 315a and an index finger below the bottom wall 315b and squeeze the walls closer together. The top wall 315a and the bottom wall 315b may be moved into contact with each other to restrict urine drainage from the opening 320. The pinching of the opening 320 is a means for restricting fluid flow. A user may control the size of the opening 320 by the force of the pinch. The opening 320 may be configured to narrow, when a user exerts a small force on the walls. Moreover, a user may exert a larger force than the force used to narrow, in order to close the opening 320 and fully prevent fluid from flowing out of the drainage member 310. Pinching closer to the distal end 317b of the body 317 may yield a narrower opening. In the embodiment shown in Figs. 3A and 3B, the proximal portion 312 may be made of either a substantially rigid material, including plastic polymer, or the flexible material. Alternatively, the drainage member 310 may be bent or kinked by folding the distal portion 314 toward the proximal portion 312.

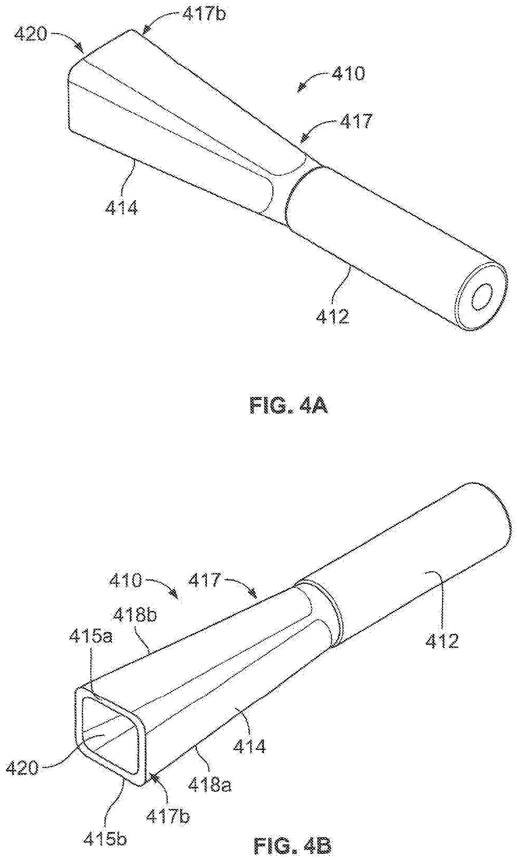

Figs. 4A and 4B show an embodiment of the drainage member 410 where the distal portion 414 of the body 417 has a truncated pyramid shape, with a four-sided opening 420. Fig. 4A shows the embodiment with the proximal portion 412 in the foreground, while Fig. 4B shows the embodiment with the distal portion 414 and the opening 420 in the foreground. As shown in Fig. 4B, the truncated pyramid shape defines sidewalls that may be moved toward each other to restrict flow. For example, the truncated pyramid shape defines a top wall 415a and a bottom wall 415b. The distal portion 414 is made from flexible material and is configured to be pinched by an external stimulus, including the force of a user's fingers, in order to narrow the opening 420. For example, a user may place a thumb over the top wall 415a and an index finger below the bottom wall 415b and squeeze the walls closer together. The walls may contact each other. The pinching of the opening 420 is a means for restricting fluid flow. A user may control the size of the opening 420 by the force of the pinch and where the pinch is placed. The opening 420 may be configured to narrow, when a user exerts a small force on the walls. Moreover, a user may exert a larger force than the force used to narrow, in order to close the opening 420 and fully prevent fluid from flowing out of the drainage member 410. Pinching closer to the distal end 417b of the body 417 may yield a narrower opening. Alternatively, the opposed surfaces 418a, 418b of the truncated pyramid may be moved toward each other to restrict urine flow. Additionally, the drainage member 410 may be kinked or bent such that the proximal portion 412 is moved in the direction of the distal portion 414. In one embodiment, the drainage member 410 may be folded over such that the proximal portion 412 may be brought into contact with the distal portion 414. In the embodiment shown in Figs. 4A and 4B, the proximal portion 412 may be made of either a substantially rigid material, including plastic polymer, or the flexible material.

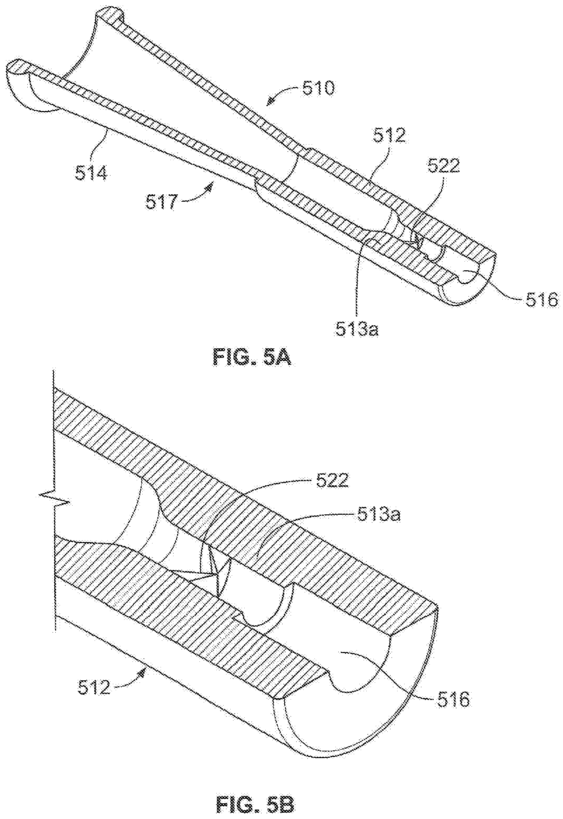

Figs. 5A and 5B show an embodiment of the drainage member 510 including a valve 522. The valve 522 may be located within the lumen 516 and may be defined by one or more inner surfaces of the body 513a. Fig.5A shows a cross-section of the drainage member 510. Fig. 5B shows a close-up view of the valve 522. The valve 522 serves to selectively restrict fluid flow. As shown, in Figs. 5A and 5B, the valve 522 may be a duckbill valve; however, any type of valve appropriate for directing fluids may be used. As shown in Fig. 5A, the valve 522 is used to direct fluid through the proximal portion 512 and into the distal portion 514. The embodiment shown in Figs. 5A and 5B allows a user to pinch on the proximal portion 512 to move the valve 522 from its relaxed state (closed valve) to its stressed state (open valve). Thus, the valve 522 opens when pressure is placed on the body 517. When the pressure is relieved, the valve 522 closes and fluid is prevented from traveling through the proximal portion 512 into the distal portion 514. As the proximal portion 512 is squeezed, by a user or an external force, it allows an increased amount of fluid to travel through the proximal portion 512. The valve 522 allows a user to selectively control and/or direct the rate of fluid as it travels through the drainage member 510. The embodiment shown in Figs. 5A and 5B has a distal portion 512 made of the flexible material, in order to facilitate movement of the valve 522.

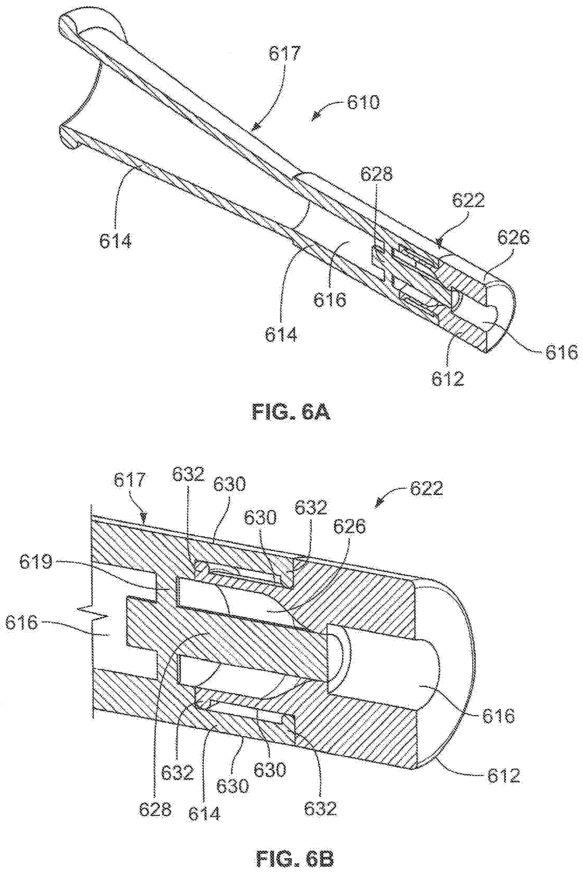

Figs. 6A and 6B show an embodiment of the drainage member 610 with a valve having a plug 628 and a seat 626. Fig. 6A shows a cross-sectional embodiment of a drainage member 610 with a valve 622, located within the lumen 616. Fig. 6B shows a close-up view of the valve 622 of Fig. 6A.

In the embodiment shown in Figs. 6A and 6B, the seat 626 is associated with the proximal portion 612 of the body 617 and the plug 628 is associated with the distal portion 614 of the body 617. The plug 628 and the seat 626 are detachably engaged. The proximal portion 612 having the seat 626 and the distal portion 614 having the plug 628 move relative to each other to engage and disengage the plug 628 from the seat 626. The plug 628 may be attached to the distal portion 614 by one or more arms 619 that extend into the lumen of the drainage member. In other embodiments, the plug 628 may be associated with the proximal portion 612 while the seat 626 may be associated with the distal portion 614.

As shown in Figs. 6A and 6B, the drainage member 610 may be configured so that at least a portion of the distal portion of the body 614 is located within and slides relative to the proximal portion of the body 612. When the distal portion 614, having the plug 628, slides into the proximal portion 612, having the seat 626, the drainage member's 610 lumen 616 is closed, restricting fluid flow. Moreover, coupling of the seat 626 and the plug 628, in one embodiment, may create a fluid-tight seal. When a user uncouples the seat 626 from the plug 628, by sliding the seat 626 and the plug 628 in opposite directions, the lumen 616 is opened, allowing fluid to pass through the proximal portion 612 to the distal portion 614.

Though the embodiment shown illustrates the distal portion 614 located within the proximal portion 612, other embodiments may feature at least a portion of the proximal portion 612 of the body 617 located within and sliding relative to the distal portion 614 of the body 617. Moreover, though one plug 628 and one seat 626 are shown in Figs. 6A and 6B, a plurality of plugs 628 and seats 626 may be used in other embodiments.

Furthermore, as shown in Fig. 6B, the drainage member 610 may include stops 630 to limit the relative movement between the proximal 612 and distal 614 portions of the body 617. The stops 630 include shoulders 632 on the proximal portion 612 and on the distal portion 614 of the body 617.

At least one of the proximal portion 612 and the distal portion 614 may be comprised of the flexible material. Furthermore, in a preferred embodiment, the plug 628 and seat 626 are comprised of a polymer which may be easily coupled and uncoupled; this polymer may include PVC plastic and styrene.

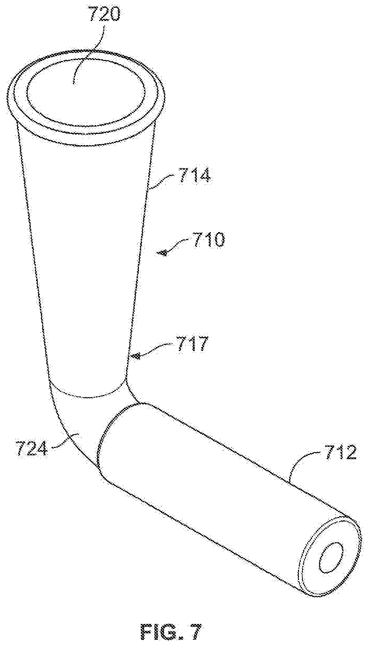

Fig. 7 shows an embodiment of the drainage member 710 wherein the distal end portion 714 of the body 717 extends at an angle from the proximal end portion 712. The distal end portion 714 may extend from the proximal end portion 712 at an angle that is less than 180 degrees. The distal end portion 714 extending at an angle less than 180 degrees may assist the user in bending or kinking the drainage member 710. For example, the less than 180 degree angle may make it easier for a user to bend or kink the drainage member 710, because the distal end portion 714 is initially closer to the proximal end portion 712 and in position that is may be more conducive to bending for some users.

In the illustrated embodiment, the drainage member 710 has a generally L- shaped configuration wherein the distal end portion 714 extends at a 90-degree angle to the proximal end portion 712. The drainage member 710 includes a flexible middle section 724 that may be bent when the proximal and distal end portions are moved toward each other. The middle section 724 is positioned between the proximal end portion 712 and the distal end portion 714 of the body 717. The body 717 may be a single component or it may be separate components wherein any appropriate manner, including friction fit and adhesive fit, may be used to connect the middle section 724 with the proximal 712 and distal 714 portions.

Furthermore, at least a portion of the proximal portion 712, the distal portion 714, and the middle section 724 may be comprised of flexible, elastic material. The flexible, elastic material may include an elastomer, rubber, or any other suitable material which may be manipulated by a user. The flexible, elastic material may be pinched or kinked to restrict urine drainage.

The elastomeric material of the middle section 724 may also allow a user to rotate the proximal portion 712 and distal portion 714 relative to one another to a broad range of other angles. The angled configuration facilitated by manipulating the middle section 724 is a means for restricting fluid flow and prevents fluid from spilling out of the drainage member opening 720.

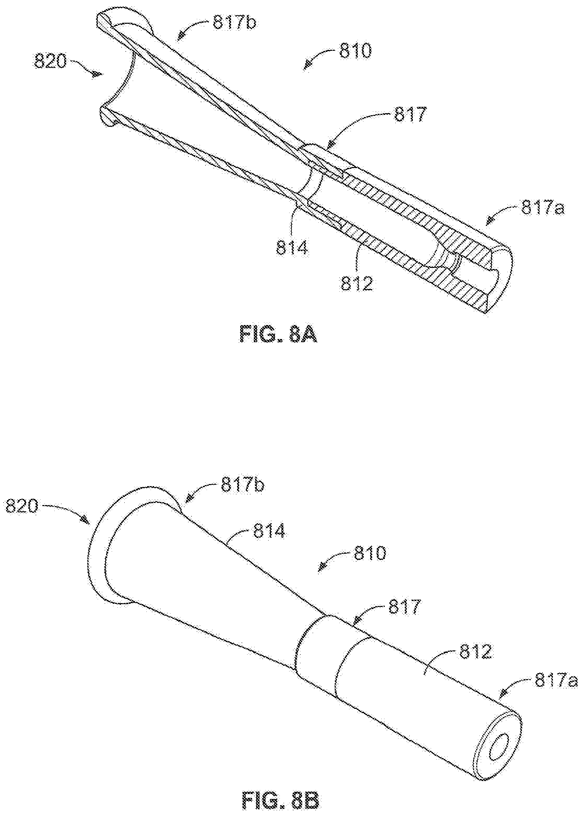

Figs. 8A and 8B show an embodiment of the drainage member 810 wherein the proximal portion 812 of the body 817 includes a rigid material and the distal portion 814 of the body 817 includes a flexible material. Fig. 8A shows a cross-sectional view of the drainage member 810. Figure 8B shows a perspective view of the drainage member 810.

The flexible material of the distal portion 814 may comprise an elastomer with lower tensile (yield) strength than the flexible material of the prior embodiments, in order to facilitate restriction of fluid flow when a weaker external force is applied. The materials of this embodiment of the drainage member 810 allow a user, perhaps one with more limited dexterity or a weak grip, to gain a steadier grasp of the proximal portion 812 and proximal end 817a, because the rigidity of the proximal portion enhances stability. Similarly, the flexibility of the distal portion 814 allows a user to more easily close or kink the distal portion 814 and distal end 817b and restrict fluid from flowing through the opening 820. The rigid material on the proximal portion 812 may include a polymer such as a high-density polyethylene or polypropylene, while the proximal portion may be made from PVC or Thermoplastic Elastomer. The embodiment shown in Figs. 8A and 8B provide a drainage member for controlling, restricting, and/or blocking fluid flow when a user exerts a lesser force.

The embodiments disclosed herein are for the purpose of providing a description of the present subject matter, and it is understood that the subject matter may be embodied in various other forms and combinations not shown in detail. It is noted that the scope of protection of the current invention is solely defined by the appended claims.

## Claims

1. A urinary catheter, comprising:
a catheter shaft (111) having a proximal end portion (111a) and a distal end portion (111b);
a drainage member (210) comprising a body (217) having a proximal end portion (212) and a distal end portion (214), the body includes an inner surface (213a) and an outer surface (213b), the inner surface (213a) defines a lumen (216) extending from the proximal end portion (212) of the body (217) through a drainage opening (220) in the distal end portion (214), the proximal end portion (212) of the body (217) being located at the distal end portion (111b) of a catheter shaft (111) such that the lumen (220) of the body (217) is in fluid communication with a drainage lumen (111c) of the catheter shaft (111); **characterized in that**,
the body (217) including a weakened portion that allows for folding the distal portion (214) of the body (217) toward the proximal end portion (212) or twisting of the distal portion (214) relative to the proximal end portion (212) to selectively restrict urine drainage from the drainage opening (220).

2. The urinary catheter of claim 1, wherein the weakened portion comprises one or more notches (218a) in the outer surface (213b) of the body (217), wherein the one or more notches allow the body to be bent or kinked to restrict urine drainage.

3. The urinary catheter of claim 2, wherein the one or more notches include a notch (218a) shaped like a semi-circle.

4. The urinary catheter of claim 2, wherein the body (217) has a middle and the one or more notches (218a) include a notch (218a) located in the middle of the body (217).

5. The urinary catheter of claim 2, wherein the one or more notches (218b) comprise a groove extending at least partially around the body.

6. The urinary catheter of claim 2, wherein in the one or more notches (218b) comprise a circumferential groove extending around the body.

7. The urinary catheter of claim 1, wherein the body (217) has a generally round cross-section.

8. The urinary catheter of claim 1, wherein the body (217) is comprised of a flexible material.

## Patentansprüche

1. Urinkatheter, umfassend:
einen Katheterschaft (111), der einen proximalen Endabschnitt (111a) und einen distalen Endabschnitt (111b) aufweist;
ein Drainageelement (210), das einen Körper (217) umfasst, der einen proximalen Endabschnitt (212) und einen distalen Endabschnitt (214) aufweist, wobei der Körper eine Innenfläche (213a) und eine Außenfläche (213b) beinhaltet, wobei die Innenfläche (213a) ein Lumen (216) definiert, das sich von dem proximalen Endabschnitt (212) des Körpers (217) durch eine Drainageöffnung (220) in dem distalen Endabschnitt (214) erstreckt, wobei sich der proximale Endabschnitt (212) des Körpers (217) an dem distalen Endabschnitt (111b) eines Katheterschafts (111) befindet, sodass das Lumen (220) des Körpers (217) in Flüssigkeitsverbindung mit einem Drainagelumen (111c) des Katheterschafts (111) steht; **dadurch gekennzeichnet, dass**
der Körper (217) einen verdünnten Abschnitt beinhaltet, der ein Falten des distalen Abschnitts (214) des Körpers (217) in Richtung des proximalen Endabschnitts (212) oder ein Verdrehen des distalen Abschnitts (214) relativ zu dem proximalen Endabschnitt (212) ermöglicht, um die Drainage von Urin aus der Drainageöffnung (220) selektiv zu begrenzen.

2. Urinkatheter nach Anspruch 1, wobei der verdünnte Abschnitt eine oder mehrere Aussparungen (218a) in der Außenfläche (213b) des Körpers (217) umfasst, wobei die eine oder mehreren Aussparungen ermöglichen, dass der Körper gebogen oder geknickt wird, um die Drainage von Urin zu begrenzen.

3. Urinkatheter nach Anspruch 2, wobei die eine oder mehreren Aussparungen eine halbkreisförmige Aussparung (218a) beinhalten.

4. Urinkatheter nach Anspruch 2, wobei der Körper (217) eine Mitte aufweist und die eine oder mehreren Aussparungen (218a) eine Aussparung (218a) beinhalten, die sich in der Mitte des Körpers (217) befindet.

5. Urinkatheter nach Anspruch 2, wobei die eine oder mehreren Aussparungen (218b) eine Nut umfassen, die sich mindestens teilweise um den Körper herum erstreckt.

6. Urinkatheter nach Anspruch 2, wobei die eine oder mehreren Aussparungen (218b) eine Umfangsnut umfassen, die sich um den Körper herum erstreckt.

7. Urinkatheter nach Anspruch 1, wobei der Körper (217) einen allgemein runden Querschnitt aufweist.

8. Urinkatheter nach Anspruch 1, wobei der Körper (217) aus einem flexiblen Material besteht.

## Revendications

1. Cathéter urinaire, comprenant :
un tube de cathéter (111) ayant une partie d'extrémité proximale (111a) et une partie d'extrémité distale (111b) ;
un élément de drainage (210) comprenant un corps (217) ayant une partie d'extrémité proximale (212) et une partie d'extrémité distale (214), le corps comprenant une surface interne (213a) et une surface externe (213b), la surface interne (213a) délimitant une lumière (216) se prolongeant à partir de la partie d'extrémité proximale (212) du corps (217) à travers une ouverture de drainage (220) ménagée dans la partie d'extrémité distale (214), la partie d'extrémité proximale (212) du corps (217) étant située au niveau de la partie d'extrémité distale (111b) d'un tube de cathéter (111), de sorte que la lumière (220) du corps (217) soit en communication fluidique avec une lumière de drainage (111c) du tube de cathéter (111) ;
**caractérisé en ce que**,
le corps (217) comprenant une partie affaiblie qui permet de plier la partie distale (214) du corps (217) vers la partie d'extrémité proximale (212) ou de tordre la partie distale (214) par rapport à la partie d'extrémité proximale (212) pour restreindre sélectivement le drainage de l'urine depuis l'ouverture de drainage (220).

2. Cathéter urinaire selon la revendication 1, dans lequel la partie affaiblie comprend une ou plusieurs encoches (218a) dans la surface externe (213b) du corps (217), dans lequel la ou les encoches permettent au corps d'être replié ou tortillé pour restreindre le drainage de l'urine.

3. Cathéter urinaire selon la revendication 2, dans lequel la ou les encoches comprennent une encoche (218a) en forme de demicercle.

4. Cathéter urinaire selon la revendication 2, dans lequel le corps (217) a un milieu et la ou les encoches (218a) comprennent une encoche (218a) située au milieu du corps (217).

5. Cathéter urinaire selon la revendication 2, dans lequel la ou les encoches (218b) comprennent une rainure se prolongeant au moins en partie autour du corps.

6. Cathéter urinaire selon la revendication 2, dans lequel la ou les encoches (218b) comprennent une rainure circonférentielle se prolongeant autour du corps.

7. Cathéter urinaire selon la revendication 1, dans lequel le corps (217) a une section transversale généralement arrondie.

8. Cathéter urinaire selon la revendication 1, dans lequel le corps (217) est constitué d'un matériau flexible.
